(19) Europäisches Patentamt
European Patent Office
Office européen des brevets

(11) **EP 1 524 009 B1**

(12) **FASCICULE DE BREVET EUROPEEN**

(45) Date de publication et mention
de la délivrance du brevet:
**07.03.2007 Bulletin 2007/10**

(51) Int Cl.:
***A61N 1/365*** *(2006.01)*      ***A61N 1/36*** *(2006.01)*

(21) Numéro de dépôt: **04292422.5**

(22) Date de dépôt: **12.10.2004**

(54) **Dispositif médical implantable actif comprenant des moyens d'ajustement de la fréquence maximale de stimulation ventriculaire en fonction de l'état hémodynamique du patient**

Aktive implantierbare medizinische Vorrichtung mit Mitteln zur Abstimmung der maximalen Frequenz der ventrikulären Stimulation in Abhängigkeit des hämodynamischen Zustandes eines Patienten

Active implantable medical device comprising means for ajusting the maximal frequency of ventrical stimulation according to the hemodynamic state of the patient

(84) Etats contractants désignés:
**AT BE BG CH CY CZ DE DK EE ES FI FR GB GR HU IE IT LI LU MC NL PL PT RO SE SI SK TR**

(30) Priorité: **16.10.2003 FR 0312106**

(43) Date de publication de la demande:
**20.04.2005 Bulletin 2005/16**

(73) Titulaire: **ELA MEDICAL**
**92541 Montrouge (FR)**

(72) Inventeur: **Dal Molin, Renzo**
**92320 Chatillon (FR)**

(74) Mandataire: **Dupuis-Latour, Dominique et al**
**SEP Pagenberg & Associés**
**14 boulevard Malesherbes**
**75008 Paris (FR)**

(56) Documents cités:
**US-A- 3 897 789          US-B1- 6 408 209**
**US-B1- 6 473 644**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Dans ces dispositifs, la fréquence de stimulation ventriculaire est variable, soit en assurant le suivi du rythme atrial par le ventricule, soit en fonction d'un paramètre recueilli par un capteur.

Dans ce dernier cas, les capteurs utilisés peuvent être de trois types :

- capteur d'effort : il s'agit d'un capteur mesurant un paramètre à prépondérance physiologique, le plus souvent par mesure de la ventilation-minute (d'où la dénomination courante de "capteur MV"), ou encore par mesure de la saturation d'oxygène dans le sang, de la température, etc. Un tel capteur fournit une représentation adéquate des besoins métaboliques du patient, selon que le patient est au repos, à l'exercice, en récupération après effort, etc.
- capteur d'activité : il s'agit généralement d'un accéléromètre intégré au stimulateur (d'où la dénomination usuelle de "capteur G"), destiné à détecter rapidement un changement de la posture ou de la dynamique du patient porteur de l'appareil, en particulier pour détecter des débuts de phase d'effort révélés par une augmentation importante des mouvements de ce patient.
- capteur hémodynamique : il s'agit dans ce cas d'opérer un asservissement à partir d'un signal représentatif du débit sanguin.

Les capteurs d'effort, également appelés capteurs physiologiques, délivrent un signal reflétant bien les besoins métaboliques réels du patient, mais avec un temps de réponse relativement long et une moindre sensibilité aux faibles efforts.

En revanche, les capteurs d'activité sont des capteurs à temps de réponse très court, mais mesurant un paramètre purement mécanique (l'accélération), de nature non physiologique, donc manquant de spécificité : un tel capteur ne permet pas, par exemple, de distinguer entre un réel début d'effort et des vibrations ou des mouvements subis de manière purement passive, par exemple dans une automobile, sans que le patient ne développe aucun effort particulier.

Les stimulateurs asservis utilisent l'un ou l'autre de ces types de capteur pour ajuster en permanence divers paramètres tels que fréquence de stimulation, délai atrioventriculaire (DAV), ou encore délai interventriculaire dans le cas d'une stimulation biventriculaire. Il existe également des stimulateurs combinant les deux types de capteurs, de manière à éviter les inconvénients propres à chacun.

Les algorithmes de pilotage des stimulateurs prévoient par ailleurs un paramètre connu sous l'appellation de "fréquence maximale" ou "$F_{max}$", qui est la fréquence maximale de stimulation ventriculaire, applicable notamment lorsqu'il s'agit d'assurer le suivi du rythme atrial par le ventricule : $F_{max}$ est alors la limite supérieure à laquelle le stimulateur peut synchroniser une stimulation ventriculaire sur chaque détection auriculaire en mode DDD. Ce paramètre $F_{max}$ sert notamment à plafonner la fréquence de stimulation calculée par des algorithmes tels que les fonctions de lissage ou d'asservissement ; dans un stimulateur asservi, $F_{max}$ sert à faire correspondre la dynamique du capteur aux valeurs extrêmes que peut prendre la fréquence de stimulation.

Dans un stimulateur double chambre, la fréquence maximale est également utilisée comme valeur de référence, comparée à la fréquence atriale détectée afin de limiter la fréquence de stimulation ventriculaire lorsque le rythme atrial dépasse $F_{max}$, par exemple en appliquant un mode de fonctionnement dit "mode Wenckebach".

La fréquence maximale est généralement programmée une fois pour toutes à une valeur déterminée par le praticien, principalement en fonction de l'âge du patient, avec éventuellement pondération par la capacité d'effort de celui-ci et/ou la présence d'une cardiopathie ou cardiomyopathie.

Il a également été déjà proposé de faire varier cette fréquence maximale de façon contrôlée au cours du temps, comme cela est par exemple décrit dans le EP-A-1 059 099 (Ela Médical), où cette fréquence est automatiquement et progressivement relevée au fil du temps en fonction de l'amélioration de l'état hémodynamique du patient.

Un mécanisme d'ajustement automatique de la fréquence maximale a également été proposé par le US-A-6 119 040 (Chirife), qui décrit un stimulateur du type asservi par un capteur d'activité (accéléromètre ou composant analogue) inclus dans le boîtier du stimulateur. Pour compenser le fait qu'un tel capteur d'asservissement n'est pas corrélé aux besoins métaboliques du patient, ce document propose de rendre variable la fréquence maximale en ajustant cette dernière de façon automatique en fonction d'un paramètre physiologique. Ainsi, une augmentation importante de la fréquence de stimulation en réponse à une situation d'activité détectée par l'accéléromètre, n'est permise que s'il y a confirmation d'une augmentation importante des besoins métaboliques, ce qui permet de rendre un peu plus spécifique le stimulateur asservi, en lui ajoutant au surplus un paramètre de sécurité important.

Dans ce document, le paramètre physiologique utilisé pour régler la fréquence maximale est la période de prééjection (PEP) ventriculaire, à savoir l'intervalle de temps compris entre la détection d'un début de cycle cardiaque (spontané ou stimulé) et le début de l'éjection ventriculaire : pendant cet intervalle de temps, le ventricule se contracte mais son volume ne change pas (contraction isovolumique), seule la pression à l'intérieur du ventricule augmente. La PEP prend fin dès que les valves aortique et pulmonaire s'ouvrent, ce qui a pour conséquence l'éjection du sang dans les artères, avec réduction corrélative du volume des ventricules, qui se poursuit jusqu'à la fin de la diastole.

Dans ce document, la PEP est évaluée à partir d'une mesure de bioimpédance intracardiaque : ce paramètre donne en effet une image dynamique de la contraction du myocarde et l'analyse des variations d'impédance permet de caractériser l'évolution des phases systolique et diastolique, et donc la durée de la PEP. Une augmentation du rythme cardiaque qui ne serait pas associée à un raccourcissement correspondant de la PEP est considérée comme inadéquate ou excessive par rapport aux besoins physiologiques du patient, ce qui permet de compenser ou, à tout le moins, de limiter les effets propres du caractère non physiologique du capteur d'activité utilisé pour l'asservissement du stimulateur.

L'un des buts de l'invention est de proposer un autre mécanisme d'adaptation de la fréquence maximale.

Il a en effet été constaté que si l'on fixe la fréquence maximale à une valeur donnée, préprogrammée, ce réglage ne prend pas en compte l'état hémodynamique général du patient, encore moins son évolution au cours du temps, par exemple dans le cas d'une amélioration - ou, au contraire, d'une aggravation - de cet état.

En effet, si la fréquence cardiaque est trop élevée, le remplissage du coeur ne peut plus s'effectuer de façon satisfaisante, et par conséquent le volume d'éjection chute. Il en est ainsi dans les situations de tachycardie ou de fibrillation, mais également d'application d'impulsions de stimulation à une fréquence trop élevée, due à un mauvais réglage de la fréquence maximale par rapport à l'état du patient.

Il existe ainsi une fréquence charnière à partir de laquelle le gain en débit sanguin obtenu par augmentation de la fréquence stimulée est perdu par la diminution du volume d'éjection. Il est donc important de ne pas dépasser cette fréquence, sous peine d'une diminution du débit cardiaque.

L'idée de base de la présente invention consiste à adapter la fréquence maximale de manière que la fréquence de stimulation ne dépasse pas ce point où la caractéristique débit/fréquence devient décroissante, ceci en réajustant si nécessaire la fréquence maximale pour tenir compte de l'évolution de cette caractéristique, qui dépend elle-même de l'état hémodynamique général du patient.

Plus précisément, le débit est évalué par une mesure de bioimpédance intracardiaque, typiquement transvalvulaire, transseptale ou intraventriculaire, techniques en elles-mêmes connues.

Il est en effet apparu aux inventeurs que, sous des conditions strictes de temps, de rythme cardiaque et d'état du capteur d'asservissement dont peut être pourvu le dispositif, les variations quotidiennes des paramètres hémodynamiques moyens qui seront obtenues par une mesure de bioimpédance intracardiaque sont représentatives de l'évolution de l'état du coeur du patient. Un paramètre hémodynamique typique est le débit cardiaque, ou un paramètre qui lui est étroitement corrélé, tel que la fraction d'éjection. Plus précisément, la mesure de bioimpédance transvalvulaire droite est représentative de l'évolution du coeur droit, tandis que la mesure de bioimpédance transseptale est représentative de l'évolution des coeurs gauche et droit.

Ces variations reflètent également, de manière indirecte, ce qui se passe sur les poumons, le coeur gauche et les tissus oxygénés du patient, du fait du retentissement général sur l'organisme du débit sanguin dans le coeur droit. Ainsi, dans le cas d'un stimulateur double chambre, si l'évolution de l'indice moyen révèle une détérioration de l'état hémodynamique du patient mais que l'activité cardiaque reste satisfaisante, le praticien pourra soupçonner une insuffisance pulmonaire. Document US-A-6408209 décrit un système médical implantable comme décrit dans le préambule de la revendication 1.

À cet effet, l'invention propose un dispositif médical implantable actif du type général décrit dans le US-A-6 119 040 précité, c'est-à-dire comprenant : des moyens de délivrance d'impulsions de stimulation sur au moins un site d'une cavité cardiaque ; des moyens de pilotage du rythme de délivrance des impulsions de stimulation ; des moyens pour limiter à une fréquence maximale le rythme de délivrance des impulsions de stimulation ; des moyens de mesure d'une bioimpédance intracardiaque ; et des moyens d'ajustement de fréquence maximale, pour modifier la valeur de la fréquence maximale en fonction de la bioimpédance intracardiaque mesurée.

Selon l'invention, les moyens d'ajustement comprennent : des moyens pour évaluer un paramètre représentatif du débit cardiaque à partir du signal délivré par les moyens de mesure de bioimpédance intracardiaque ; des moyens pour commander une variation prédéterminée de la fréquence de délivrance des impulsions de stimulation ; des moyens pour évaluer la variation corrélative du débit cardiaque ; et des moyens pour ajuster la valeur de la fréquence maximale en fonction de la variation du débit cardiaque ainsi évaluée.

Les moyens d'ajustement sont très avantageusement des moyens opérant de manière itérative, aptes à commander des variations prédéterminées successives de la fréquence de stimulation, et à évaluer à chaque itération la variation corrélative du débit cardiaque.

Les moyens d'ajustement mettent alors fin à l'itération lorsque, pour des incrémentations successives de la fréquence de stimulation, la variation corrélative du débit cardiaque devient inférieure à un seuil donné ce seuil pouvant notamment être un pourcentage donné d'augmentation du débit cardiaque, rapporté à un pourcentage donné d'augmentation de la fréquence de stimulation ; ils ajustent ensuite la valeur de la fréquence maximale à la valeur de la fréquence de stimulation qui avait été appliquée à l'avant-dernière itération. Le seuil en question peut être un seuil fixe, notamment un pourcentage donné d'augmentation du débit cardiaque, rapporté à un pourcentage donné d'augmentation de la fréquence de stimulation, ou bien un seuil variable, ajusté dynamiquement en fonction de la fréquence cardiaque ou du débit cardiaque.

Avantageusement, les moyens d'ajustement peuvent aussi mettre fin de manière anticipée à l'itération si l'incrémentation de la fréquence de stimulation conduit celle-ci à dépasser une valeur limite prédéterminée.

Les moyens pour évaluer le paramètre représentatif du débit cardiaque peuvent être des moyens opérant à partir de valeurs de pics d'impédance diastolique et d'impédance systolique déterminées par les moyens de mesure de bioimpédance intracardiaque, ou bien opérant par intégration du signal de mesure de la bioimpédance intracardiaque, cette intégration étant opérée entre des pics successifs d'impédance diastolique et/ou d'impédance systolique.

Les moyens peuvent en outre permettre, après avoir ajusté la valeur de la fréquence maximale, d'ajuster une valeur de délai auriculo-ventriculaire et/ou une valeur de délai interventriculaire. Dans ce cas, ils comprennent avantageusement, comme précédemment, des moyens pour commander une variation prédéterminée du délai auriculo-ventriculaire et/ou du délai interventriculaire, des moyens pour évaluer la variation corrélative du débit cardiaque, et des moyens pour ajuster la valeur du délai auriculo-ventriculaire et/ou du délai interventriculaire en fonction de la variation du débit cardiaque ainsi évaluée. Ces moyens peuvent notamment opérer de manière itérative, en commandant des variations prédéterminées successives du délai auriculo-ventriculaire et/ou du délai interventriculaire, et en évaluant à chaque itération la variation corrélative du débit cardiaque.

On va maintenant décrire un exemple de mise en oeuvre du dispositif de l'invention, en référence aux dessins annexés.

La figure 1 est un diagramme montrant l'évolution de l'impédance intracardiaque en fonction du temps au cours de deux cycles cardiaques successifs.

La figure 2 illustre la caractéristique donnant le débit cardiaque en fonction de la fréquence de stimulation.

La figure 3 est un organigramme illustrant la manière dont la fréquence maximale est, selon l'invention, ajustée au plus près du maximum de la caractéristique de la figure 2.

La présente invention concerne, de façon générale, un stimulateur cardiaque (ou un défibrillateur, cardioverteur ou dispositif multisite), éventuellement asservi sur un capteur d'effort (capteur physiologique), typiquement un capteur de ventilation-minute. La ventilation-minute est un facteur représentatif des besoins métaboliques instantanés du patient, et elle est évaluée par une mesure de bioimpédance transpulmonaire, c'est-à-dire opérée entre le coeur et le boîtier du stimulateur, situé dans le haut du thorax.

L'invention propose essentiellement de modifier la fréquence maximale de stimulation (ci-après $F_{max}$) en fonction de l'amélioration ou non du débit cardiaque chez le patient porteur du dispositif, ce débit étant évalué par une mesure de bioimpédance intracardiaque.

La mesure d'une bioimpédance intracardiaque est une technique en elle-même connue.

Ainsi, le EP-A-1 116 497 (ELA Médical) décrit une manière d'effectuer une mesure dynamique de bioimpédance intracardiaque permettant d'évaluer les volumes diastolique et systolique, et d'obtenir ainsi une indication du débit cardiaque et donc de la fraction d'éjection. Le signal obtenu est utilisé pour piloter la fréquence cardiaque et/ou le délai atrioventriculaire dans le sens de la maximisation du débit ; il est également proposé d'utiliser ce paramètre pour piloter le délai interventriculaire, dans le cas d'un stimulation biventriculaire.

Plus précisément, ce document décrit une technique de mesure de la bioimpédance transvalvulaire (entre oreillette et ventricule situés d'un même côté du coeur) par une configuration tripolaire, avec injection d'une impulsion de courant entre un site atrial et un site ventriculaire, et recueil d'un potentiel différentiel entre un site atrial et un site ventriculaire, avec l'un des sites commun à l'injection et au recueil, un site propre d'injection et un site propre de recueil. Le courant injecté est un courant de faible amplitude, insuffisant pour exciter les cellules cardiaques.

Le EP-A-1 138 346 (ELA Médical) décrit un autre type de mesure de bioimpédance, qui est une bioimpédance transseptale, c'est-à-dire entre un site situé d'un côté du coeur et un site situé de l'autre côté du coeur, avec une configuration transseptale oblique (entre un ventricule et l'oreillette située du côté opposé) ou transseptale interventriculaire (entre les deux ventricules).

Cette technique permet, ici encore, de délivrer une valeur représentative de la fraction d'éjection, bien que le signal soit plus faible que dans le cas de la mesure d'une bioimpédance transvalvulaire, et soit également influencé par l'impédance propre des tissus du septum. Par ailleurs, tandis que la bioimpédance transvalvulaire est corrélée au débit du coeur droit, la bioimpédance transseptale, oblique ou interventriculaire, est corrélée au débit du coeur gauche.

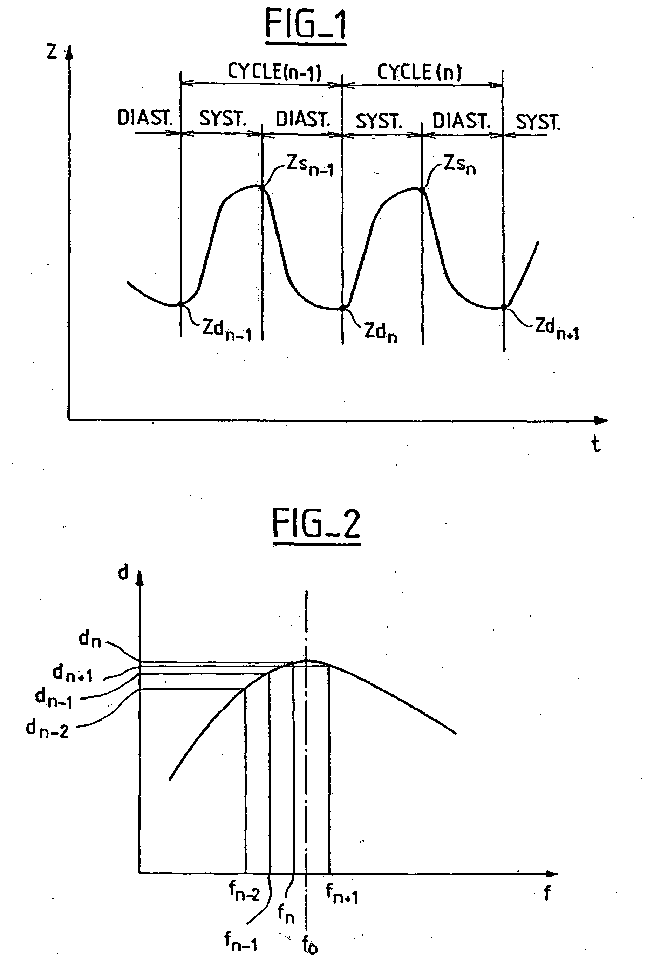

Sur la figure 1 on a illustré la variation de l'impédance intracardiaque Z, plus précisément d'une impédance transvalvulaire, au cours de deux cycles cardiaques successifs. De façon générale, pour les besoins de l'invention, les mesures de bioimpédance intracardiaque peuvent se faire par mesure bipolaire, tripolaire, quadripolaire ou plus, selon des techniques en elles-mêmes connues qui ne seront pas exposées ici plus en détail.

Les valeurs d'impédance $Zd_{n-1}$, $Zs_{n-1}$, $Zd_n$, $Zs_n$, $Zd_{n+1}$, etc. qui correspondent aux débuts des phases systoliques et diastoliques successives sont détectées par le changement du sens de variation de la courbe d'impédance Z.

La différence entre l'impédance systolique $Zs$ et l'impédance diastolique $Zd$ donne une valeur corrélée au volume d'éjection ; la loi liant le volume et l'impédance intracardiaque n'étant pas nécessairement linéaire, une compensation peut s'avérer nécessaire.

D'autres techniques sont possibles pour évaluer le volume d'éjection, par exemple par intégration de l'aire comprise entre :

- l'impédance diastolique initiale $Zd_n$ et l'impédance systolique $Zs_n$ d'un même cycle (cycle n),
- l'impédance systolique $Zs_n$ et l'impédance diastolique finale $Zd_{n+1}$ d'un même cycle (cycle n),
- les impédances systoliques $Zs_{n-1}$ et $Zs_n$ de deux cycles successifs, ou
- les impédances diastoliques $Zd_{n-1}$ et $Zd_n$ de deux cycles successifs.

Le volume d'éjection ainsi déterminé peut être éventuellement moyenné sur un nombre préprogrammé de cycles cardiaques.

À partir de cette valeur éventuellement moyennée du volume d'éjection, il est possible de déterminer le débit cardiaque :

$$\text{débit (en l/min)} = \text{volume d'éjection (en l)} \times \text{fréquence cardiaque (en cpm)}$$

L'invention propose, en utilisant les mesures de débit cardiaque ainsi obtenues, de faire varier la fréquence de stimulation f de manière à approcher au plus près le sommet de la caractéristique débit/fréquence, illustrée figure 2, et à donner à la fréquence maximale $F_{max}$ la valeur de la fréquence $f_0$ correspondant à ce maximum, ou une valeur la plus proche possible de cette dernière.

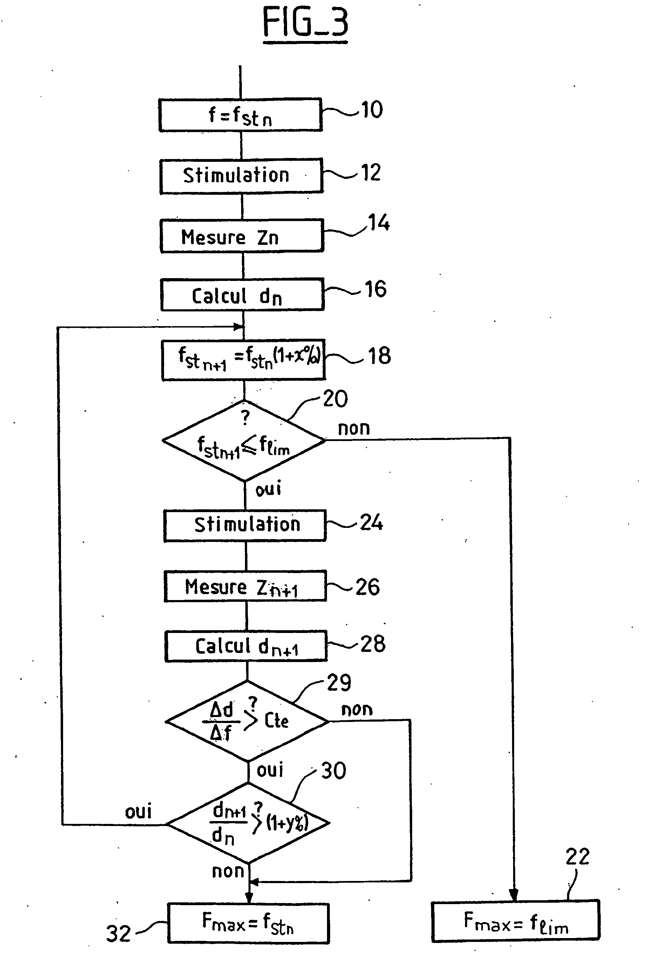

Le mécanisme correspondant est un mécanisme itératif, procédant par approximations successives, illustré par l'organigramme de la figure 3.

Pour une fréquence de stimulation donnée (étape 10), le dispositif produit une stimulation (étape 12) et mesure les variations d'impédance intracardiaque correspondantes (étape 14). De la manière indiquée plus haut, le débit correspondant $d_n$ est alors évalué (étape 16).

Le dispositif augmente ensuite la fréquence de stimulation d'une certaine quantité, par exemple de $x$ % (étape 18). En variante, au lieu d'une augmentation proportionnelle, on pourrait également augmenter la fréquence de stimulation par pas constants.

L'algorithme vérifie ensuite (étape 20) que la fréquence de stimulation ainsi augmentée ne dépasse une valeur limite $f_{lim}$, préalablement fixée par le praticien au moment de la programmation du dispositif, et ce de façon inconditionnelle. En effet, comme la fréquence maximale $F_{max}$ est recalculée à intervalles réguliers au lieu d'être un paramètre fixe, il est important qu'elle ne puisse pas atteindre des valeurs qui, en tout état de cause, seraient considérées comme excessives et dangereuses pour le patient.

Si cette fréquence limite $f_{lim}$ est atteinte ou dépassée, il est mis fin à l'algorithme et l'on donne à $F_{max}$ la valeur de la fréquence limite $f_{lim}$ (étape 22).

Dans le cas contraire, le dispositif produit une stimulation avec la fréquence ainsi accrue de $x$ % (étape 24), mesure la nouvelle impédance intracardiaque correspondante (étape 26) et évalue la nouvelle valeur du débit (étape 28).

En 29, le dispositif vérifie que le taux d'accroissement $\Delta f$ est positif, c'est-à-dire que l'on se trouve sur une partie croissante de la courbe : si $\Delta d/\Delta f$ est positif, on se dirige vers un maximum, tandis que si $\Delta d/\Delta f$ est nul on se trouve au maximum et si $\Delta d/\Delta f$ est négatif ce maximum a été dépassé ; pour conserver une marge d'erreur, on fixe comme critère $\Delta d/\Delta f >$ un seuil constant.

Ensuite (étape 30) l'algorithme détermine si, pour cette augmentation de $x$ % de la fréquence, le débit cardiaque a augmenté d'au moins $y$ % ($x$ et $y$ étant des valeurs programmables définies à l'avance).

Dans l'affirmative, ceci signifie que le couple fréquence/débit se place sur un point de la caractéristique de la figure 2 se trouvant sur la partie croissante de cette caractéristique, avec une pente encore relativement importante, et que ce point est donc encore assez éloigné du maximum (tel est le cas des fréquences $f_{n-2}$ et $f_{n-1}$ sur la figure 2).

Dans le cas contraire, c'est-à-dire si le débit cardiaque augmente de moins de y % lorsque la fréquence de simulation a augmenté de x %, on considère que la fréquence $f_0$ correspondant au maximum de la caractéristique a été dépassée, ou que l'on se situe dans une plage très proche de ce maximum (cas des fréquences $f_n$ et $f_{n+1}$ de la figure 2).

Il est alors mis fin à l'itération, et la fréquence maximale $F_{max}$ est fixée à la valeur de l'avant-dernière fréquence de stimulation, c'est-à-dire la fréquence qui avait été appliquée avant la dernière augmentation de $x$ % (étape 32).

Bien entendu, comme on l'a indiqué plus haut, à chaque itération on vérifie que la fréquence de stimulation ne dépasse en aucun cas la fréquence limite $F_{lim}$ fixée à l'avance, quand bien même le maximum de la caractéristique ne serait pas atteint.

Après qu'il ait mis fin à l'algorithme, c'est-à-dire après les étapes 22 ou 32, le dispositif exécutera à nouveau les étapes de l'organigramme de la figure 3 lorsque diverses conditions prédéterminées auront été vérifiées. Ceci peut notamment se présenter :

i) lorsqu'un capteur d'effort (capteur MV) ou un capteur d'activité (capteur G) détecte une situation d'effort exercé par le patient ou, hors capteur, lorsque le rythme atrial augmente d'un certain pourcentage au-delà un seuil donné, ou ii) lorsqu'aucune variation significative du rythme n'a été détectée au bout d'un délai programmable, par exemple au bout de trois heures.

Par ailleurs, l'invention peut être appliquée à la commande d'un paramètre autre que la fréquence maximale, en particulier le délai AV (délai auriculo-ventriculaire), ou le délai W (délai interventriculaire, dans le cas d'une stimulation biventriculaire).

Tout ce qui vient d'être dit ci-dessus à propos de l'ajustement de la fréquence maximale de stimulation ventriculaire $F_{max}$ peut donc être directement transposé, *mutatis mutandis,* à l'ajustement d'un délai AV ou d'un délai W (en variante, aussi bien qu'en complément, de l'ajustement de $F_{max}$).

Ainsi, pour piloter le délai W dans le sens d'une maximisation du débit, le dispositif doit rechercher en permanence l'optimum de la fréquence de stimulation, du délai W et du délai AV. Pour ce faire, la fréquence maximale $F_{max}$ est d'abord optimisée, puis le délai AV, en recherchant un optimum par approches successives, suivant la technique exposée plus haut ; enfin l'optimum du délai W est recherché (l'ordre de ces opérations pouvant être modifié). La recherche de l'optimum se fait en diminuant ou en augmentant la valeur de façon à déterminer le sens optimal de variation, puis en affinant la valeur ; chaque fois que la fréquence, ou le débit, changent, une nouvelle optimisation est lancée.

**Revendications**

1. Un dispositif médical implantable actif, notamment un stimulateur cardiaque, défibrillateur, cardioverteur et/ou dispositif multisite, comprenant :

- des moyens de délivrance d'impulsions de stimulation sur au moins un site d'une cavité cardiaque,
- des moyens de pilotage du rythme de délivrance des impulsions de stimulation,
- des moyens pour limiter à une fréquence maximale ($F_{max}$) le rythme de délivrance des impulsions de stimulation,
- des moyens de mesure d'une bioimpédance intracardiaque (Z), et
- des moyens d'ajustement de fréquence maximale, pour modifier la valeur de la fréquence maximale ($F_{max}$) en fonction de la bioimpédance intracardiaque (Z) mesurée,

dispositif **caractérisé en ce que** les moyens d'ajustement comprennent :

· des moyens pour évaluer un paramètre représentatif du débit cardiaque à partir du signal délivré par les moyens de mesure de bioimpédance intracardiaque (Z),
· des moyens pour commander une variation prédéterminée ($x$ %) de la fréquence (f) de délivrance des impulsions de stimulation,
· des moyens pour évaluer la variation corrélative ($y$ %) du débit cardiaque, et
· des moyens pour ajuster la valeur de la fréquence maximale ($F_{max}$) en fonction de la variation du débit cardiaque ainsi évaluée.

2. Le dispositif de la revendication 1, dans lequel les moyens d'ajustement sont des moyens opérant de manière itérative, aptes à commander des variations prédéterminées successives de la fréquence de stimulation, et à évaluer à chaque itération la variation corrélative du débit cardiaque.

3. Le dispositif de la revendication 2, dans lequel les moyens d'ajustement sont aptes à mettre fin à l'itération lorsque, pour des incrémentations successives de la fréquence de stimulation, la variation corrélative du débit cardiaque devient inférieure à un seuil.

4. Le dispositif de la revendication 3, dans lequel ledit seuil est un seuil fixe donné.

**5.** Le dispositif de la revendication 4, dans lequel ledit seuil est un pourcentage donnée ($y$ %) d'augmentation du débit cardiaque, rapporté à un pourcentage donné ($x$ %) d'augmentation de la fréquence de stimulation.

**6.** Le dispositif de la revendication 3, dans lequel ledit seuil est un seuil variable, ajusté dynamiquement en fonction de la fréquence cardiaque ou du débit cardiaque.

**7.** Le dispositif de la revendication 3, dans lequel les moyens d'ajustement sont aptes, après avoir mis fin à l'itération, à ajuster la valeur de la fréquence maximale ($F_{max}$) à la valeur de la fréquence de stimulation qui avait été appliquée à l'avant-dernière itération.

**8.** Le dispositif de la revendication 2, dans lequel les moyens d'ajustement sont aptes à mettre fin à l'itération si l'incrémentation de la fréquence de stimulation conduit celle-ci à dépasser une valeur limite prédéterminée.

**9.** Le dispositif de la revendication 1, dans lequel les moyens pour évaluer un paramètre représentatif du débit cardiaque sont des moyens opérant à partir de valeurs de pics d'impédance diastolique ($Z_d$) et d'impédance systolique ($Z_s$) déterminées par les moyens de mesure de bioimpédance intracardiaque.

**10.** Le dispositif de la revendication 1, dans lequel des moyens pour évaluer un paramètre représentatif du débit cardiaque sont des moyens opérant par intégration du signal de mesure de la bioimpédance intracardiaque, cette intégration étant opérée entre des pics successifs d'impédance diastolique ($Z_d$) et/ou d'impédance systolique ($Z_s$).

**11.** Le dispositif de la revendication 1, dans lequel les moyens d'ajustement sont des moyens aptes, après avoir ajusté la valeur de la fréquence maximale, à ajuster en outre une valeur de délai auriculo-ventriculaire et/ou une valeur de délai interventriculaire.

**12.** Le dispositif de la revendication 11, dans lequel les moyens d'ajustement comprennent des moyens pour commander une variation prédéterminée du délai auriculo-ventriculaire et/ou du délai interventriculaire, des moyens pour évaluer la variation corrélative du débit cardiaque, et des moyens pour ajuster la valeur du délai auriculo-ventriculaire et/ou du délai interventriculaire en fonction de la variation du débit cardiaque ainsi évaluée.

**13.** Le dispositif de la revendication 12, dans lequel les moyens d'ajustement sont des moyens opérant de manière itérative, aptes à commander des variations prédéterminées successives du délai auriculo-ventriculaire et/ou du délai interventriculaire, et à évaluer à chaque itération la variation corrélative du débit cardiaque.

**Claims**

**1.** An active implantable medical device, in particular a pacemaker, defibrillator, cardioverter and/or multisite device, comprising:

- means for delivering stimulation pulses to at least one site of a cardiac cavity,
- means for controlling the delivery rhythm of the stimulation pulses,
- means for limiting the delivery rhythm of the stimulation pulses to a maximum frequency (Fmax),
- means for measuring an intracardiac bio-impedance (Z), and
- means for adjusting the maximum frequency in order to modify the value of the maximum frequency (Fmax) as a function of the measured intracardiac bio-impedance (Z),

the device being **characterised in that** the adjustment means comprise:

■ means for evaluating a parameter representative of the cardiac output based upon the signal delivered by the means for measuring the intracardiac bio-impedance (Z),
■ means for controlling a predetermined variation (x %) of the delivery frequency (f) of the stimulation pulses,
■ means for evaluating the correlative variation (y %) of the cardiac output, and
■ means for adjusting the value of the maximum frequency (Fmax) as a function of the such evaluated variation of the cardiac output.

**2.** The device of claim 1, wherein the adjustment means are means operating in an iterative way, able to control successive predetermined variations of the stimulation frequency, and to evaluate with each iteration the correlative variation of the cardiac output.

3. The device of claim 2, wherein the adjustment means are able to terminate the iteration when, for successive increments of the stimulation frequency, the correlative variation of the cardiac output becomes lower than a threshold.

4. The device of claim 3, wherein said threshold is a given fixed threshold.

5. The device of claim 4, wherein said threshold is a given percentage (y %) of increase in the cardiac output, in relation to a given percentage (x %) of increase in the stimulation frequency.

6. The device of claim 3, wherein said threshold is a variable threshold, dynamically adjusted as a function of the heart rate or the cardiac output.

7. The device of claim 3, wherein the adjustment means are able to adjust the value of the maximum frequency (Fmax) to the value of the stimulation frequency that had been applied at the penultimate iteration, after having terminated the iteration.

8. The device of claim 2, wherein the adjustment means are able to terminate the iteration if the incrementation of the stimulation frequency leads to it exceeding a predetermined limit value.

9. The device of claim 1, wherein the means for evaluating a parameter representative of the cardiac output are means operating on the basis of peak values of diastolic impedance (Zd) and of systolic impedance (Zs) determined by the means for measuring the intracardiac bio-impedance.

10. The device of claim 1, wherein the means for evaluating a parameter representative of the cardiac output are means operating by integrating the measured intracardiac bio-impedance signal, this integration being done between successive peaks of diastolic impedance (Zd) and/or of systolic impedance (Zs).

11. The device of claim 1, wherein the adjustment means are means able to furthermore adjust a value of the atrio-ventricular delay and/or a value of the inter-ventricular delay, after having adjusted the value of the maximum frequency.

12. The device of claim 11, wherein the adjustment means comprise means for controlling a predetermined variation of the atrio-ventricular delay and/or the inter-ventricular delay, means for evaluating the correlative variation of the cardiac output, and means for adjusting the value of the atrio-ventricular delay and/or inter-ventricular delay as a function of the such evaluated variation of the cardiac output.

13. The device of claim 12, wherein the adjustment means are means operating in an iterative way, able to control successive predetermined variations of the atrio-ventricular delay and/or inter-ventricular delay, and to evaluate with each iteration the correlative variation of the cardiac output.

**Patentansprüche**

1. Aktive implantierbare medizinische Vorrichtung, insbesondere Herzschrittmacher, Defibrillator, Kardioverter und/oder Multisite-Vorrichtung, mit:

- Mitteln zur Abgabe von Stimulationsimpulsen an wenigstens einen Herzkammersitz,
- Mitteln zur Steuerung des Rhythmus der Abgabe der Stimulationsimpulse,
- Mitteln zur Begrenzung des Rhythmus der Abgabe der Stimulationsimpulse auf eine Maximalfrequenz (Fmax),
- Mitteln zur Messung einer intrakardialen Bioimpedanz (Z), und
- Mitteln zur Anpassung der Maximalfrequenz, um den Wert der Maximalfrequenz (Fmax) in Abhängigkeit der gemessenen intrakardialen Bioimpedanz (Z) zu verändern,

Vorrichtung, die **dadurch gekennzeichnet ist, dass** die Mittel zur Anpassung folgendes umfassen:

■ Mittel, um anhand des durch die Mittel zur Messung der intrakardialen Bioimpedanz (Z) gelieferten Signals einen für das Herzminutenvolumen repräsentativen Parameter zu bestimmen,

■ Mittel zur Steuerung einer vorbestimmten Veränderung (x %) der Abgabefrequenz (f) der Stimulationsimpulse,
■ Mittel zur Bestimmung der korrelativen Veränderung (y %) des Herzminutenvolumens, und
■ Mittel zur Anpassung des Wertes der Maximalfrequenz (Fmax) in Abhängigkeit der Veränderung des so bestimmten Herzminutenvolumens.

2.  Vorrichtung nach Anspruch 1, bei welcher die Mittel zur Anpassung Mittel sind, die auf iterative Weise arbeiten, welche geeignet sind, aufeinander folgende vorbestimmte Veränderungen der Stimulationsfrequenz zu steuern, und bei jeder Iteration die korrelative Veränderung des Herzminutenvolumens zu bestimmen.

3.  Vorrichtung nach Anspruch 2, bei welcher die Mittel zur Anpassung geeignet sind, die Iteration zu beenden, wenn bei aufeinander folgenden Inkrementierungen der Stimulationsfrequenz die korrelative Veränderung des Herzminutenvolumens geringer als eine Schwelle wird.

4.  Vorrichtung nach Anspruch 3, bei welcher die Schwelle eine gegebene festgelegte Schwelle ist.

5.  Vorrichtung nach Anspruch 4, bei welcher die Schwelle ein gegebener Prozentsatz (y %) der Erhöhung des Herzminutenvolumens ist, bezogen auf einen gegebenen Prozentsatz (x %) der Erhöhung der Stimulationsfrequenz.

6.  Vorrichtung nach Anspruch 3, bei welcher die Schwelle eine veränderbare Schwelle ist, die dynamisch in Abhängigkeit der Herzfrequenz oder des Herzminutenvolumens angepasst wird.

7.  Vorrichtung nach Anspruch 3, bei welcher die Mittel zur Anpassung geeignet sind, nachdem sie die Iteration beendet haben, den Wert der Maximalfrequenz (Fmax) an den Wert der Stimulationsfrequenz anzupassen, die bei der vorletzten Iteration angewandt wurde.

8.  Vorrichtung nach Anspruch 2, bei welcher die Mittel zur Anpassung geeignet sind, die Iteration zu beenden, falls die Inkrementierung der Stimulationsfrequenz dazu führt, dass diese einen vorbestimmten Grenzwert überschreitet.

9.  Vorrichtung nach Anspruch 1, bei welcher die Mittel zur Bestimmung eines für das Herzminutenvolumen repräsentativen Parameters Mittel sind, die aufgrund von Spitzenwerten der diastolischen Impedanz (Zd) und der systolischen Impedanz (Zs) arbeiten, welche durch die Mittel zur Messung der intrakardialen Bioimpedanz bestimmt werden.

10. Vorrichtung nach Anspruch 1, bei welcher Mittel zur Bestimmung eines für das Herzminutenvolumen repräsentativen Parameters Mittel sind, die durch Integration des Messsignals der intrakardialen Bioimpedanz arbeiten, wobei diese Integration zwischen aufeinander folgenden Spitzen der diastolischen Impedanz (Zd) und/oder der systolischen Impedanz (Zs) erfolgt.

11. Vorrichtung nach Anspruch 1, bei welcher die Mittel zur Anpassung Mittel sind, die geeignet sind, nachdem der Wert der Maximalfrequenz angepasst wurde, außerdem einen Wert der atrio-ventrikulären Verzögerung und/oder einen Wert der interventrikulären Verzögerung anzupassen.

12. Vorrichtung nach Anspruch 11, bei welcher die Mittel zur Anpassung Mittel zur Steuerung einer vorbestimmten Veränderung der atrio-ventrikulären Verzögerung und/oder der interventrikulären Verzögerung umfassen, Mittel, um die korrelative Veränderung des Herzminutenvolumens zu bestimmen, und Mittel, um den Wert der atrio-ventrikulären Verzögerung und/oder der interventrikulären Verzögerung in Abhängigkeit der Veränderung des so bestimmten Herzminutenvolumens anzupassen.

13. Vorrichtung nach Anspruch 12, bei welcher die Mittel zur Anpassung Mittel sind, die auf iterative Weise arbeiten, welche geeignet sind, aufeinander folgende vorbestimmte Veränderungen der atrio-ventrikulären Verzögerung und/oder der interventrikulären Verzögerung zu steuern, und bei jeder Iteration die korrelative Veränderung des Herzminutenvolumens zu bestimmen.

## FIG_1

## FIG_2

# FIG_3

$$f = f_{st\,n} \quad \sim 10$$

Stimulation $\sim 12$

Mesure $Z_n$ $\sim 14$

Calcul $d_n$ $\sim 16$

$$f_{st\,n+1} = f_{st\,n}(1+x\%) \quad \sim 18$$

$$f_{st\,n+1} \leqslant f_{lim} \;?\quad \sim 20$$
non

oui

Stimulation $\sim 24$

Mesure $Z_{n+1}$ $\sim 26$

Calcul $d_{n+1}$ $\sim 28$

$$\frac{\Delta d}{\Delta f} \overset{?}{>} Cte \quad \sim 29$$
non

oui $\sim 30$

$$\frac{d_{n+1}}{d_n} \overset{?}{>} (1+y\%)$$
oui

non

$$F_{max} = f_{st\,n} \quad 32$$

$$F_{max} = f_{lim} \quad 22$$